# EUROPEAN PATENT APPLICATION

(11) **EP 2 357 197 A1**
(43) Date of publication of application: **17.08.2011**
(21) Application number: 10001569.2
(22) Date of filing: 16.02.2010
(51) Int. Cl.: C07K 16/08, G01N 33/569

(54) **Anti-HPV E7 antibodies**

(71) Applicant: Österreichische Akademie der Wissenschaften, 1010 Wien (AT); Austria Wirtschaftsservice Gesellschaft m.b.H., 1030 Wien (AT)
(72) Inventor: Jansen-Dürr, Pidder, 6020 Innsbruck (AT); Zwerschke, Werner, 6020 Innsbruck (AT); Pircher, Haymo, 6170 Zirl (AT); Ehehalt, Daniela, 6020 Innsbruck (AT); Lener, Barbara, 6020 Innsbruck (AT); Dreier, Kerstin, 6020 Innsbruck (AT)
(74) Representative: Schrell, Andreas

(57) **Abstract**

The present invention relates to monoclonal anti-HPV (human papillomavirus) E7 antibodies capable of specifically recognising an epitope of the C-terminal or the N-terminal region of a HPV E7 protein, diagnostic compositions and kits comprising said antibodies as well as methods for immunohistochemical and ELISA-based diagnosis of HPV infections utilizing said antibodies.

SEQUENCE LISTING

<110>  AWS

<120>  E7 Antikörper

<130>  202185 EP

<160>  5

<170>  PatentIn version 3.5

<210>  1
<211>  5
<212>  PRT
<213>  HPV 16 and 18 subtypes E7


<220>
<221>  misc_feature
<222>  (4)..(4)
<223>  Xaa can be any naturally occurring amino acid

<400>  1

Val Cys Pro Xaa Cys
1               5


<210>  2
<211>  10
<212>  PRT
<213>  HPV 16 subtypes E7


<220>
<221>  misc_feature
<222>  (2)..(5)
<223>  Xaa can be any naturally occurring amino acid

<220>
<221>  misc_feature
<222>  (9)..(9)
<223>  Xaa can be any naturally occurring amino acid

<400>  2

Gly Xaa Xaa Xaa Xaa Val Cys Pro Xaa Cys
1               5                   10


<210>  3
<211>  13
<212>  PRT
<213>  HPV16-E7

<400>  3

Gly Thr Leu Gly Ile Val Cys Pro Ile Cys Ser Gln Lys
1               5                   10


<210>  4
<211>  10
<212>  PRT
<213>  HPV 18 subtype E7

<220>
<221>  misc_feature
<222>  (5)..(5)
<223>  Xaa can be any naturally occurring amino acid

<220>
<221>  misc_feature
<222>  (8)..(9)
<223>  Xaa can be any naturally occurring amino acid

<400>  4

Phe Val Cys Pro Xaa Cys Ala Xaa Xaa Gln
1               5                   10


<210>  5
<211>  9
<212>  PRT
<213>  HPV18-E7

<400>  5

Lys Ala Thr Leu Gln Asp Ile Val Leu
1               5

## Description

The present invention relates to monoclonal anti-HPV (human papillomavirus) E7 antibodies capable of specifically recognising an epitope of the C-terminal or the N-terminal region of a HPV E7 protein, diagnostic compositions and kits comprising said antibodies as well as methods for immunohistochemical and ELISA-based diagnosis of HPV infections utilizing said antibodies.

Sexually transmitted infections with human papillomaviruses (HPVs) are the main etiological factor for cervical cancer. Over thirty-five HPV genotypes, that can infect epithelial squamous and glandular cells in the cervical mucosa, have been described. On the basis of epidemiological and biochemical data, the double stranded DNA HPVs are divided into high-risk HPVs associated with squamous intraepithelial lesions with a high potential for progression to invasive squamous cell carcinoma, and low-risk HPVs associated with benign hyperplasia. Infections by high-risk genotypes have been detected in almost all neoplastic lesions of the cervix and at least 15 high-risk types of HPV have been associated with cervical cancer. HPV 16 is the most prevalent genotype worldwide with an incidence in squamous cell carcinoma of approximately 55 %, followed by HPV 18 (approximately 17 %) and HPV 45 (4-9 %).

The persistence of oncogenic HPV is necessary for the development of cervical precancer and cancer. However, the factors that determine viral persistence and tumorigenic progression are not fully understood. The initial events of cervical carcinogenesis after viral infection depend on the fact that high-risk HPV types undergo specific changes that abrogate the transcriptional control of viral gene expression in the infected keratinocytes. Inactivation of these cellular control functions permits deregulated transcription of the early viral genes E6 and E7, thereby triggering cell proliferation, inhibition of apoptosis, reprogramming of differentiation and chromosomal instability. These changes can support the integration of episomal HPV genomes into chromosomes of the host cell, and contribute to further overexpression of the viral genes E6 and E7, resulting in an increase of the E7 oncoprotein levels during early steps of cervical carcinogenesis.

That the viral oncoproteins E6 and E7 are crucial during carcinogenesis was further proven by the fact that high-risk E7 protein, in cooperation with high-risk E6, can efficiently immortalize human primary keratinocytes in vitro. Moreover, the consistent overexpression of the E6 and E7 oncogenes is required to induce and to maintain the transformed phenotype of cervical cancer cells.

Thus, overexpression of E7 oncoproteins of carcinogenic HPV types is a characteristic feature of cervical cancer. This conclusion is supported by a recent study where affinity-purified antibodies against high-risk HPV E7 proteins were used to detect the E7 oncoproteins of HPV 16, 18 and 45 in biopsies from invasive cervical squamous cell carcinoma patients (Ressler et al., 2007, Clin Cancer Res, 13:7067-7072), indicating that the high-risk E7 oncoproteins of these major high-risk HPV types are expressed continuously in invasive cervical carcinoma.

At present, clinical cervical cancer screening is mainly based on the cytological assessment of cells contained in cervical smears, referred to as Pap Smears (or Papanicolaou test; Papanicolaou, 1942, Science, 95:438-439), that are routinely taken from women participating in screening programs. The result of the cytological analysis is dependent on detection of abnormal cells by experienced pathologists. Although the implementation of this simple and efficient assay has helped to considerably reduce the mortality of cervical cancer, Pap Smear diagnosis is still characterized by a high rate of false-positive and false-negative results (Foucar, 2005, Semin Diagn Pathol, 22(2):147-155).

In an alternative approach, detection of high-risk HPV DNA has been introduced into clinical practice. In this setting, a sensitive DNA detection assay (e.g., Hybrid capture 2TM; Digene Inc., USA) is applied to determine the presence of high-risk HPV DNA in patient samples. Although this assay is well suited to detect infections by papillomaviruses of the high-risk type, it cannot discriminate between transient infections, which spontaneously regress in most cases, and the onset of cervical cancer. More recently, diagnostic systems have been developed that detect the presence of E6/E7 mRNA in cervical smears, as a surrogate marker for the expression of the viral oncoproteins E6 and E7. However, it has been shown that the level of E6/E7 mRNA is not significantly changing during carcinoma progression, suggesting that RNA detection may not be the best tool for cervical cancer screening (Hafner et al., 2008, Oncogene, 27(11):1610-1617). In accordance with this notion, the underlying HPV infection could not be detected by PCR analysis in all examined tumors by Ressler et al., 2007.

Therefore, the detection of E7 protein seems to be the superior diagnostic tool. Various antibodies are already known in the art, but they either display low sensitivity or specificity or are described to cross-react with the E7 proteins of various HPV types. Also, as polyclonal antibodies can only be produced in a limited quantity by one animal, there are batch-to-batch differences. The difficulty to produce highly specific and sensitive monoclonal antibodies against E7 proteins is mainly due to the low immunogenicity of E7 proteins.

WO 07/059492 for example, discloses methods, assays and kits for the detection of HPV DNA or proteins, employing inter alia monoclonal and polyclonal antibodies against the HPV 16 E7 protein, capable of reliably detecting the rather high amount of around 1 µg of recombinant HPV 16 E7 protein.

Accordingly, the technical problem underlying the present invention is to overcome the aforementioned difficulties and to provide highly specific and more sensitive means for a cost-efficient, rapid and reliable diagnosis of high-risk HPV infections.

The present invention solves its underlying problem according to the teaching of the independent claims. Accordingly, the present invention provides monoclonal antibodies against the E7 proteins of high-risk HPV types, in particular provides a monoclonal anti-HPV (human papillomavirus) E7 antibody, which is capable of specifically recognising an epitope of the C-terminal region of a HPV E7 protein.

The term "antibody" as employed herein preferably refers to a full immunoglobulin, like an IgG, IgA, IgM, IgD or IgE but in another embodiment may also refer to a fragment of an antibody, like an F(ab), F(abc), Fv, Fc or F(ab)₂ or a fused antibody, a fused antibody fragment or another derivative of the antibody of the present invention as long as it still displays the specificity and sensitivity of the full immunoglobulin of the present invention.

The term "specificity" or "specifically recognising/binding" is understood to refer to the property of an antibody to specifically recognise or bind to preferably only one specific epitope and preferably shows no or substantially no cross-reactivity to another epitope. Preferably "specificity" or "specifically recognising/binding" means that the antibody specifically recognises or binds to preferably only one specific epitope and preferably shows no or substantially no cross-reactivity to another epitope, wherein said epitope is unique for one protein, such that the antibody shows no or substantially no cross-reactivity to another epitope and another protein.

The term "sensitivity" as employed herein refers to the detection limit of an antibody, which is preferably low, i. e. at least below a concentration of 1 µg of the protein to be detected.

In general, the term "epitope" refers to an antigenic region of a given protein which consists of 4 to 50, preferably 5 to 15, preferably 8 to 11 amino acids, or more. This antigenic region or epitope is specifically recognised by the antigen binding site(s) of the antibody.

In the context of the present invention, the term "C-terminal region" is understood to refer to the at most 30, 35, 40, 45, 50, 55 or 60 C-terminal amino acids of the HPV E7 protein.

In a preferred embodiment, the monoclonal anti-HPV E7 antibody specifically recognises an epitope of the zinc finger domain of the C-terminal region of the HPV E7 protein, preferably said epitope is a conformational epitope.

The monoclonal antibodies according to the present invention are preferably used to detect native, i. e. non-denatured HPV E7 proteins.

In the context of the present invention, the term "conformational epitope" refers to a three-dimensional epitope which the antibody specifically recognises or binds to as opposed to a linear epitope. Linear epitopes are determined solely by the amino acid sequence, whereas conformational epitopes are determinded bythe three-dimensional surface features, i. e. the tertiary structure.. Therefore, the amino acids of a conformational epitope defining the antigenicity of a protein do not necessarily have to be consecutive amino acids of the protein but can be distant from each other in the primary protein sequence to form the conformational epitope, only if the protein is folded in its tertiary structure. Mostly, such conformational epitopes cannot be recognised by the antibody, if the protein is denatured, i. e. not folded into its tertiary structure.

HPV E7 proteins contain a zinc finger domain in their C-terminal part, which forms a highly ordered structure, mainly due to 4 cystein residues. These cystein residues coordinate zinc and thereby represent a conformational epitope. Surprisingly, it has been found that the monoclonal antibodies according to the present invention which are capable of recognising or binding to such a conformational epitope, are able to detect a HPV E7 protein in a highly specific and highly sensitive manner.

In a preferred embodiment, the monoclonal antibody is capable of specifically recognising an epitope of the C-terminal region, preferably the zinc finger domain, of the E7 protein of HPV 16 subtypes, in particular HPV types 16, 31, 33, 35, 52, 58 or of HPV 18 subtypes, in particular HPV types 18, 39, 45, 59, 68, 70 or of both HPV 16 and 18 subtypes. Preferably, the recognised epitope is a conformational epitope. Preferably, the recognised epitope comprises the amino acid sequence set forth in SEQ ID No. 1 which is a common epitope sequence motif present in epitopes of the present invention. The epitope motif characterised by SEQ ID No. 1 represents a consensus epitope motif for HPV 16 and 18 subtypes and is: Val Cys Pro Xaa Cys, with Xaa being any amino acid.

The term "consensus epitope motif" as employed herein refers to specific parts of an epitope which are shared by the E7 proteins of HPV 16 and/or 18 subtypes. Despite sharing such a consensus epitope motif, the C-terminal regions of HPV 16 and/or 18 subtypes, in particular the specific epitopes contained therein and containing this epitope motif are, due to specific amino acid sequences and/or a specific conformation of the amino acid sequence, substantially different enough, so that specific antibodies are able to specifically recognise a particular E7 protein from a specific HPV type, i. e. are capable of identifying specifically one particular HPV type.

In a preferred embodiment of the present invention, the monoclonal antibody is capable of specifically recognising an epitope of the C-terminal region, preferably the zinc finger domain, of HPV 16 subtypes, in particular 16, 31, 33, 35, 52 and 58. Preferably, the recognised epitope is a conformational epitope. Preferably, the recognised epitope comprises the amino acid sequence set forth in SEQ ID No. 2 which is a common sequence motif present in epitopes of the HPV 16 subtypes. The epitope motif characterised by the amino acid sequence of SEQ ID No. 2 represents a consensus epitope motif specific for HPV 16 subtypes, and is: Gly Xaa Xaa Xaa Xaa Val Cys Pro Xaa Cys, with Xaa being any amino acid.

In a particularly preferred embodiment of the present invention, the monoclonal antibody is capable of specifically recognising the E7 protein of HPV 16. Preferably, the recognised epitope comprises, preferably consists of, the amino acid sequence set forth in SEQ ID No. 3. The epitope characterised by the amino acid sequence of SEQ ID No. 3 represents a specific conformational epitope of the zinc finger domain in the C-terminal part of the HPV 16 E7 protein, and is: Gly Thr Leu Gly Ile Val Cys Pro Ile Cys Ser Gln Lys.

This epitope lies within a region of the E7 protein usually displaying low immunogenicity. Surprisingly, it could be shown that the monoclonal antibody recognising the epitope as given in SEQ ID No. 3 is able to specifically detect the E7 protein of HPV 16 in a highly sensitive manner and does not cross-react with E7 proteins of other HPV types. Compared to the prior art, the present monoclonal antibody is at least 100 times more sensitive with no loss of specificity. As infections with HPV 16 account for over 50 % of cervix carcinoma cases worldwide, the highly specific antibody according to the present invention is suitable to serve as a highly effective and reliable diagnostic tool. Advantageously, it could be shown that the monoclonal anti-HPV 16 E7 antibody according to the present invention is able to detect low amounts such as 1 pg of HPV 16 E7 protein in an ELISA-based assay (enzyme-linked immunosorbant assay), whereas the antibodies disclosed in WO 07/059492 could only detect concentrations of HPV 16 E7 protein of about 1 µg. The monoclonal anti-HPV 16 E7 antibody according to the present invention can therefore be used in much lower concentrations, which renders its application as a diagnostic tool also more cost-effective.

It has been shown that E7 protein levels increase from undetectable to substantial levels during carcinogenesis. With the highly sensitive monoclonal antibodies according to the invention it is possible to detect not only tumor cells of squamous cell carcinoma and adenocar-cinomas, but also cells of high-grade precancerous cervical intraepithelial neoplasia and carcinoma in situ (CIN III and CIS) as well as cervical intraepithelial glandular neoplasia grade III and adenocarcinoma in situ (CIGN III and AIS), which were reported to have a high potential to progress to invasive cancer. This might advantageously permit early diagnosis of high-grade precancerous CIN III, CIS, CIGN III and AIS before their progression to malignant tumors, as the monoclonal antibodies according to the present invention can discriminate between low grade precancerous lesions of squamous cell and glandular origin (CIN I-II, CIGN I-II) on the one hand, and high-grade precancers (CIN III, CIS, CIGN III) and cancers (CxCa, AdCa) of squamous cell and glandular origin on the other hand. Thus, the monoclonal antibodies according to the present invention can serve as a new tool to identify high-grade precancers and cancers of squamous cell and glandular origin.

Thus, the present invention preferably relates to a monoclonal antibody specifically recognising an epitope comprising the amino acid sequence of SEQ ID No. 1, 2, 3 and 4, preferably comprising or consisting of SEQ ID No. 3.

The present invention also relates to a hybridoma cell line, preferably a mammalian, most preferred a rabbit hybridoma cell line, capable of producing the above-identified monoclonal antibody according to the invention, in particular of the antibody capable to specifically recognising the E7 protein of HPV 16, preferably capable of recognising an epitope of the C-terminal region of HPV 16, in particular a conformational epitope, preferably an epitope with the amino acid sequence of SEQ ID No. 3. The present invention preferably relates to the hybridoma cell line which has the accession no. DSM ACC 3034. In a preferred embodiment the present invention also relates to the monoclonal antibody, which is obtainable from the supernatant of a hybridoma cell line according to the present invention.

The term "hybridoma cell line" as employed herein refers to a cell line obtained by fusing myeloma cells, preferably immortalized, mammalian, in particular rabbit, lymphoid cells with B-cells of the spleen of a mammal, preferably a rabbit that has been immunized with the desired antigen, i. e. a purified HPV E7 protein. Such an immortal hybridoma cell line is able to produce one specific and defined type of antibody which is secreted into the cell culture medium or supernatant. The term "monoclonal" refers to the cell line from which the antibody is obtained, wherein all the cells are clones of a single parent cell. This permits advantageously standardized production and purification procedures to obtain monoclonal antibodies of the same quality.

Furthermore, such hybridoma cell lines can be grown indefinitely in the suitable cell culture media thereby providing an infinite source of the monoclonal antibody, without batch-to-batch variations. This is particularly advantageous for an application of monoclonal antibodies in clinical diagnostic routines, as time-consuming tests of new antibody batches can be omitted and diagnostic methods produce constant and reliable results.

In a preferred embodiment of the present invention, the monoclonal antibody is capable of specifically recognising an epitope of the C-terminal region, preferably the zinc finger domain, of HPV 18 subtypes, in particular 18, 39, 45, 59, 68 and 70. Preferably, the recognised epitope is a conformational epitope. Preferably, the epitope comprises the amino acid sequence set forth in SEQ ID No. 4, which is a common sequence motif present in epitopes of the HPV 18 subtypes and is: Phe Val Cys Pro Xaa Cys Ala Xaa Xaa Gln, with Xaa being any amino acid.

The epitope motif characterised by the amino acid sequence of SEQ ID No. 4 represents a consensus epitope motif of the HPV 18 subtypes.

In another aspect, the present invention relates to a monoclonal anti-HPV E7 antibody, which is capable of specifically recognising an epitope of the N-terminal region of a human papillomavirus E7 protein. In a particularly preferred embodiment the monoclonal antibody specifically recognises an epitope of the N-terminal region of the E7 protein of HPV 18 subtypes, preferably of HPV 18, HPV 45 or both. Preferably, the antibody specifically recognising the N-terminal region of HPV 18 E7 protein shows cross-reactivity with HPV 11, 45, 56, 58, 59 and 70. Preferably, the recognised specific epitope comprises, preferably consists of, the amino acid sequence set forth in SEQ ID No. 5 and is: Lys Ala Thr Leu Gln Asp Ile Val Leu.

The term "N-terminal region" as employed herein refers to the at most 30, 35, 40, 45, 50, 55 or 60 N-terminal amino acids of the HPV E7 protein.

The present invention also relates to a hybridoma cell line, preferably a mammalian, most preferred a rabbit, hybridoma cell line, capable of producing the monoclonal antibody capable of specifically recognising an epitope of the N-terminal region of the HPV 18 E7 protein. The present invention preferably relates to the hybridoma cell line, which has the accession no. DSM ACC 3035.

In a particularly preferred embodiment, the monoclonal antibodies according to the present invention are mammalian, preferably rabbit, monoclonal antibodies (RabMabs), preferably produced by a rabbit hybridoma cell line. Due to a more sophisticated immune system, rabbits are able to produce more elaborate antibodies than mice. This advantage combined with the possibility to create rabbit hybridoma cell lines makes it possible to obtain high quality monoclonal antibodies.

For various applications the monoclonal antibodies according to the present invention may be detectably labelled themselves with a radioactive, enzymatic or fluorescent group. A variety of techniques is available for labelling biomolecules such as antibodies, which are well-known to the person skilled in the art. Commonly used labels comprise inter alia fluorochroms, like fluorescein, rhodamine, Texas Red, Cy3 or Cy5, enzymes like peroxidase, horse radish peroxidase, β-galactosidase, alkaline phosphatase or acetylcholinesterase, radioactive isotopes, digoxygenin, colloidal metals, chemi- or bioluminescent compounds. Preferably, the monoclonal antibodies according to the present invention are labelled with biotin. In another preferred embodiment, the antibodies of the present invention are detected by secondary methods, like indirect immunofluoresence. Accordingly, detectably labelled secondary antibodies may be employed to specifically detect the monoclonal antibodies according to the present invention.

In a preferred embodiment, the present invention also relates to a diagnostic composition, comprising the monoclonal antibodies according to the present invention as an active agent. Said diagnostic composition may, in a preferred embodiment, comprise the monoclonal antibody of the present invention in soluble form or liquid phase. In another preferred embodiment the present antibodies are bound, attached and/or linked to a solid support. In another preferred embodiment, the present diagnostic composition comprises the monoclonal antibodies according to the invention formulated with a diagnostically acceptable carrier, diluent, buffer or storage solution. The diagnostic composition of the present invention comprises in a preferred embodiment the monoclonal antibodies individually or in combinations according to the requirements of the intended application. Preferably, said diagnostic composition is used for the immunological, preferably immunohistological, immunofluorescence- or ELISA-based detection of HPV E7 protein in a biological sample to enable the diagnosis of HPV infections.

The present invention also relates to a diagnostic kit, comprising the monoclonal antibodies according to the invention as a first agent and other antibodies directed against HPV E7 proteins as a second agent. The antibodies used as a second agent are preferably polyclonal goat antibodies, capable of recognising the E7 proteins of high-risk HPV 16, 18, 31, 33, 35, 39, 45, 52, 56, 58 and 59.

The diagnostic kit of the present invention comprises in a preferred embodiment the monoclonal antibodies individually or in combinations according to the requirements of the intended application.

The diagnostic kit according to the present invention is preferably used for immunological, preferably immunohistochemical, immunofluorescence- or ELISA-based detection of HPV E7 protein, thereby enabling the diagnosis of HPV infections. Preferably, the diagnostic kit of the present invention further comprises optionally one or more buffers, storage solutions and/or other reagents or materials required for medical, scientific or diagnostic purposes. Furthermore, parts of the diagnostic kit can be packaged individually in vials or bottles or in combination in containers or multi-container units.

In a particularly preferred embodiment, the present invention provides an in vitro method for the immunological, preferably immunohistochemical or immunofluorescence-based diagnosis of HPV infections, comprising a) incubating a biological sample with the monoclonal antibodies according to the present invention and b) measuring and/or detecting HPV E7 protein, preferably the E7 protein of high-risk HPV types, in the biological sample by measuring and/or detecting the antibody specifically bound to the E7 protein and thereby allowing the diagnosis of a HPV infection. Advantageously, the application of the monoclonal antibodies according to the present invention in such a diagnostic method allows for a highly sensitive and reliable detection of high-risk HPV infections, as the monoclonal antibodies according to the present invention display a very high signal-to-noise ratio with negligible background. In a preferred embodiment, the monoclonal anti HPV E7 antibody capable of specifically recognising an epitope of the C-terminal or N-terminal region of the HPV E7 protein is used in this method.

The term "biological sample" as employed herein is understood to refer to any kind of biological tissue, cells and/or organs, preferably cervix biopsies or smears, preferably Pap Smears.

In yet another particularly preferred embodiment, the present invention relates to an in vitro method for the ELISA-based diagnosis of HPV infections, comprising aa) coating a support with capture antibodies directed against HPV E7 proteins, bb) adding a biological sample, preferably a cell lysate, to the coated support, cc) incubating the support with the monoclonal detection antibody according to the present invention, and dd) identifying HPV E7 protein specifically bound by the capture antibodies by measuring and/or detecting the monoclonal detection antibody specifically bound to the E7 protein and thereby allowing the diagnosis of a HPV infection, preferably high-risk HPV infection. In a preferred embodiment, the monoclonal anti HPV E7 antibody capable of specifically recognising the C-terminal or N-terminal region of the HPV E7 protein is used as detection antibody in this method.

Preferably, polyclonal goat antibodies are used as capture antibodies for the ELISA-based diagnosis method, which are capable of recognising the E7 proteins of high-risk HPV types 16, 18, 31, 33, 35, 39, 45, 52, 56, 58 and 59. As these polyclonal goat antibodies only bind E7 proteins of high-risk HPV types, the application of the anti-HPV 16 E7 antibody and the anti-HPV 18 E7 antibody as detection antibodies advantageously allows the detection of high-risk HPV types 16, 18, 45, 56, 58, 59 and 70 which together account for > 95 % of all cervical cancers.

As support or solid support ELISA-plates are preferably used but different carriers or materials may as well be chosen by the person skilled in the art.

Cell lysates to be tested with the ELISA-based method are preferably prepared from biological samples, preferably cervix biopsies or smears, preferably Pap Smears.

Further preferred embodiments are the subject matter of the subclaims.

The sequence listing shows:
SEQ ID No. 1 shows the amino acid sequence of a C-terminal consensus epitope motif of the E7 protein of HPV 16 subtypes and HPV 18 subtypes and
SEQ ID No. 2 shows the amino acid sequence of a C-terminal consensus epitope motif of the E7 protein of HPV 16 subtypes and
SEQ ID No. 3 shows the amino acid sequence of the C-terminal conformational epitope of the HPV 16 E7 protein and
SEQ ID No. 4 shows the amino acid sequence of a C-terminal consensus epitope motif of the E7 protein of HPV 18 subtypes and
SEQ ID No. 5 shows the amino acid sequence of the N-terminal epitope of the HPV 18 E7 protein.

The present invention is further illustrated by the following nonlimiting examples and the accompanying figures.

The figures show:

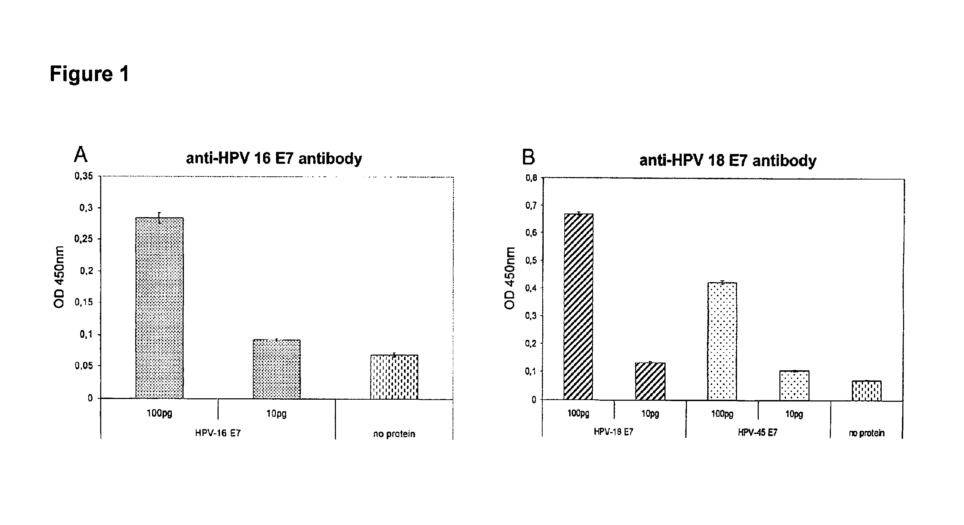

Figure 1 shows the sensitivity of the monoclonal antibodies of the present invention for the detection of recombinant E7 proteins by ELISA. ELISA plates were coated with a mixture (1:1) of goat polyclonal antibodies against HPV 16 E7 and HPV 18 E7. Then, recombinant E7 proteins were added in different concentrations. Subsequently, the monoclonal anti-HPV 16 E7 (1A) and anti-HPV 18 E7 (1 B) antibodies of the present invention were added for the detection of the recombinant proteins bound by the goat polyclonal antibodies, followed by incubation with a corresponding secondary antibody for visualization.

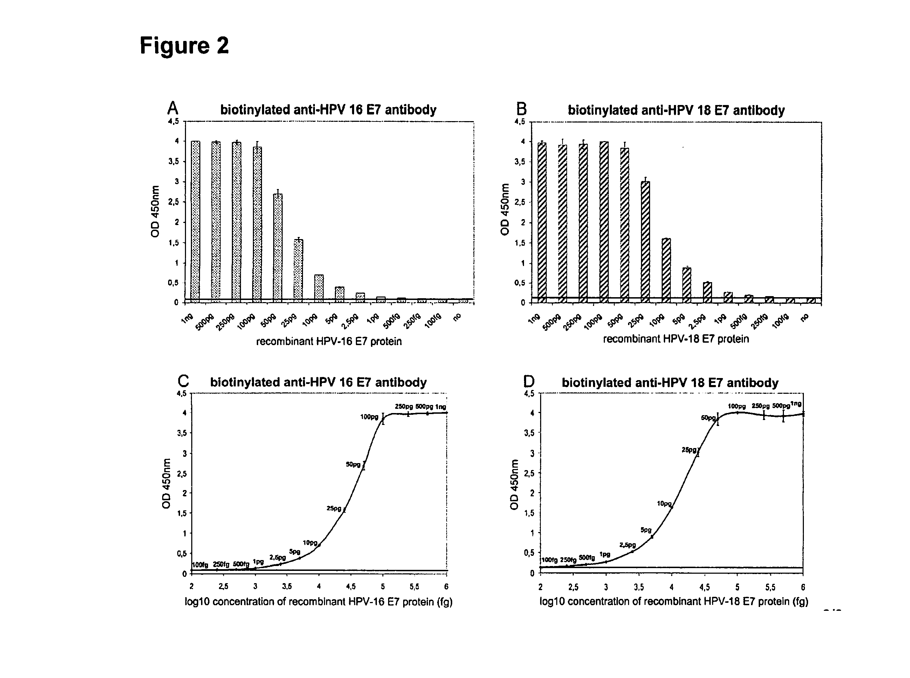

Figure 2 shows the sensitivity of the biotinylated monoclonal antibodies of the present invention for the detection of recombinant E7 proteins by ELISA (2 A, B) and the logarithmic representation of the protein concentrations detected by the antibodies of the present invention (C, D). ELISA plates were coated with a mixture (1:1) of goat polyclonal antibodies against HPV 16 E7 and HPV 18 E7. Then, recombinant E7 proteins were added in different concentrations. Subsequently, the biotinylated monoclonal anti-HPV 16 E7 (2A, C) and anti-HPV 18 E7 (2B, D) antibodies of the present invention were added for detection of the E7 proteins bound by the goat polyclonal antibodies, followed by incubation with Streptavidin-Poly-HRP-conjugate for visualization. The detection limit is defined as mean of the background signal (twelve measurements) plus three times the standard deviation.

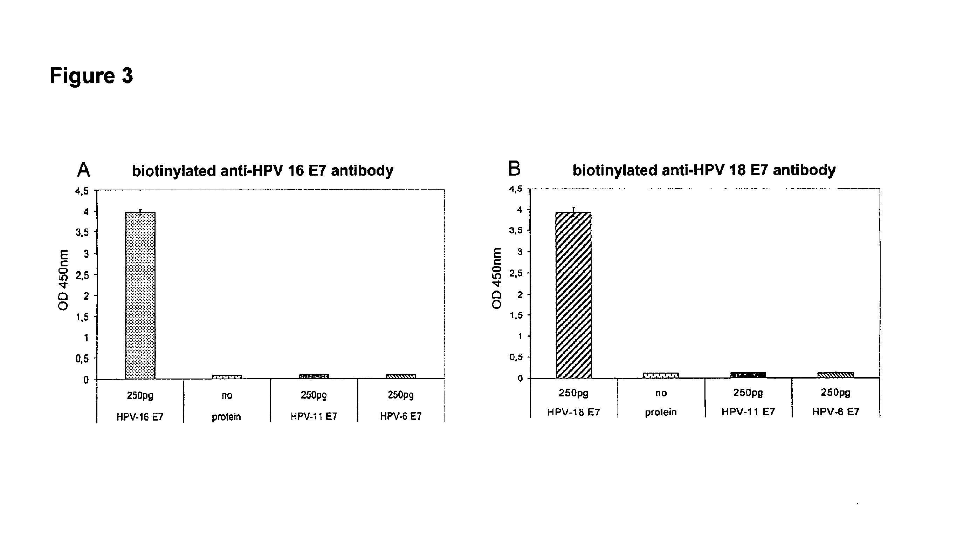

Figure 3 shows that the biotinylated monoclonal antibodies of the present invention do not detect recombinant proteins of low-risk HPV types (6, 11) in an ELISA. ELISA plates were coated with a mixture (1:1) of goat polyclonal antibodies directed against HPV 16 E7 and HPV 18 E7. Then, different recombinant E7 proteins of high-risk HPV types (16, 18) and low-risk HPV types (6, 11) were added. Subsequently, the biotinylated monoclonal anti-HPV 16 E7 (3A) and anti-HPV 18 E7 (3B) antibodies of the present invention were added for the detection of the E7 proteins bound by the goat polyclonal antibodies, followed by incubation with Streptavidin-Poly-HRP-conjugate for visualization.

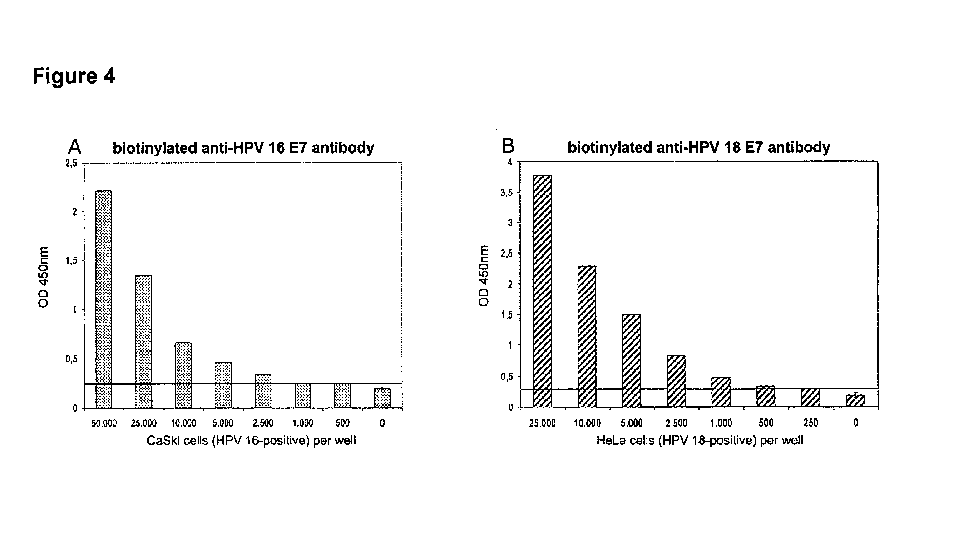

Figure 4 shows the detection of endogenous E7 protein of CaSki-cells (HPV 16 positive) (4A) and HeLa-cells (HPV 18 positive) (4B) in the background of HPV negative U-2OS-cells with the biotinylated monoclonal antibodies of the present invention by ELISA. Therefore, ELISA plates were coated with a mixture (1:1) of goat polyclonal antibodies against HPV 16 E7 and HPV 18 E7. Cell lysates with a total cell concentration of 100,000 cells per well consisting of HPV negative U-2OS-cells as background and different amounts of HPV 16 E7 positive CaSki-cells or HPV 18 E7 positive HeLa-cells were added.
   Subsequently, the biotinylated monoclonal anti-HPV 16 E7 (4A) and anti-HPV 18 E7 (4B) antibodies of the present invention were added for the detection of E7 proteins bound by the goat polyclonal antibodies, followed by incubation with Streptavidin-Poly-HRP-conjugate for visualization. Detection limit is defined as mean of the background signal (12 measurements) plus three times the standard deviation.

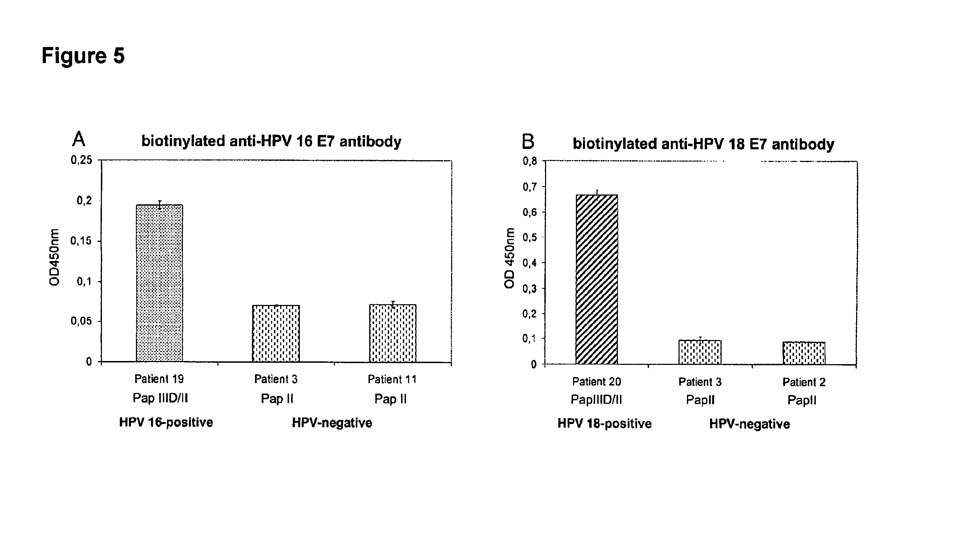

Figure 5 shows the detection of E7 protein in five clinical samples (one HPV 16 positive, one HPV 18 positive and three HPV negative) with the biotinylated monoclonal antibodies of the present invention by ELISA. ELISA plates were coated with a mixture (1:1) of goat polyclonal antibodies against HPV 16 E7 and HPV 18 E7. Lysates of the clinical samples (100 µg/well) were added. Subsequently, he biotiniylated monoclonal anti-HPV 16 E7 (5A) and anti-HPV 18 E7 (5B) antibodies of the present invention were added for the detection of E7 proteins bound by the goat polyclonal antibodies, followed by incubation with Streptavidin-Poly-HRP conjugate for visualization.

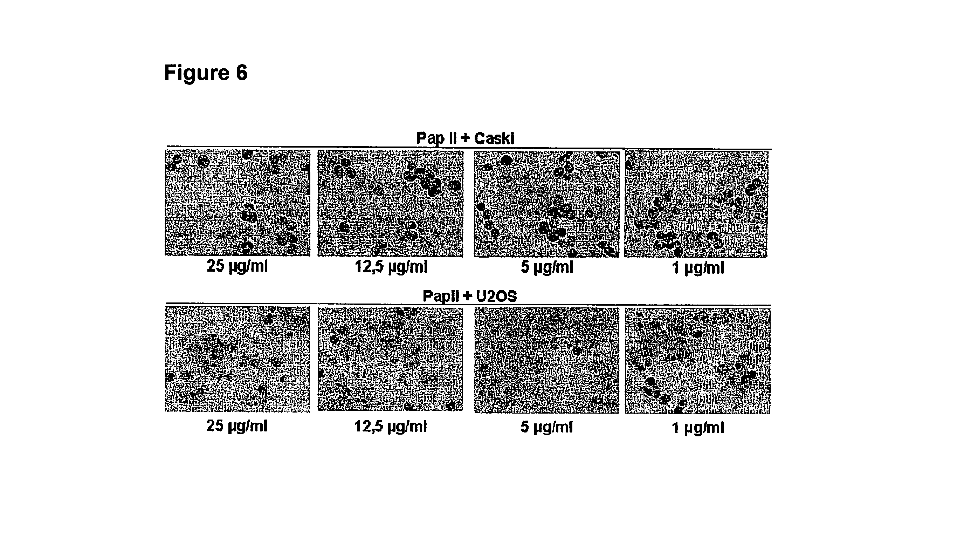

Figure 6 shows the detection of E7 protein in LBC samples form healthy women spiked with CaSki cells (HPV 16 positive) and U-20S cells (HPV negative). Liquid-based cytology was prepared from a cervical smear of a PapII proband, which did not yield any signal with anti-HPV 16 E7 antibody of the present invention. As indicated, 10,000 cells each of CaSki and U-20S were added to the sample. HPV 16 E7 positive cells were specifically detected by immunohistochemistry with anti-HPV 16 E7 antibody of the present invention over a wide range of antibody dilutions, as indicated.

### Example 1

### Generation and purification of rabbit monoclonal antibodies (RabMabs)

Purified HPV 16 and HPV 18 E7 proteins (purity > 95 %; 4 mg each) (Fiedler et al., J Gen Virol, 2005, 86, 3235-3241, and Fiedler et al., J Virol Methods, 2006, 134, 30-35) were used to immunize rabbits, and rabbit hybridoma clones were prepared. Selected hybridomas were taken in culture and supernatants (2 I each) were produced, which typically yielded 2 mg of the respective RabMab. Hybridoma supernatant was diluted with 1/3 binding buffer (20 mM sodium phosphate, pH 7) and filtered through a 0.45 µm filter. The column (HiTrapTM Protein G HP 5 ml; GE Healthcare) was washed with 5-10 column volumes of binding buffer at 5 ml/min. The filtered supernatant was applied and the column washed with 5-10 column volumes of binding buffer; eluted with 6x2 ml elution, buffer (0.1 M glycine-HCl, pH 2,7) into collection tubes with 80 µl 3 M Tris-HCl, pH 9. After elution, the column was washed with 5-10 column volumes of binding buffer followed by 5-10 column volumes of 20 % ethanol. IgG-containing fractions were pooled and dialyzed overnight at 4 °C against dialysis buffer (166 mM potassium phosphate, 83 mM glycine, pH 7.2). After dialysis, samples were concentrated using concentration tubes (Amicon Ultra, MWCO 10,000 Millipore). Finally, the sample was cleared by centrifugation (13,000 rpm, 4 °C, 10 min) and the IgG concentration was determined using OD at 280 nm. Aliquots were stored in liquid nitrogen.

Hybridoma clones producing the desired antibodies were selected after further testing and a hybridoma clone producing monoclonal anti-HPV 16 E7 antibodies has been deposited on 16^{th} of December 2009 with the DSMZ, Braunschweig, Germany, under the accession no. DSM ACC 3034 and a second hybridoma clone producing monoclonal anti-HPV 18 E7 antibodies has been deposited on 16^{th} of December 2009 with the DSMZ under the accession no. DSM ACC 3035.

### Example 2

### Cross-reactivity test for E7 antibodies

U-2OS-cells (HPV negative) were transiently transfected with vectors for overexpression of selected E7 proteins. For confocal immunofluorescence microscopy, cells were fixed in 4% (w/v) PFA/PBS and permeabilized with 0.1% (w/v) sodium citrate/0.3% (v/v) Triton X-100. After blocking with 1% (w/v) bovine serum albumin/PBS, cells were incubated for 1 h with the monoclonal anti-HPV 16 E7 or anti-HPV 18 E7 of the present invention (25 µg/ml) at room temperature and analyzed by confocal microscopy.

The results showed that the anti-HPV 16 E7 antibody of the present invention did not cross-react with E7 proteins of other HPV types and the anti-HPV 18 E7 antibody of the present invention was able to detect E7 proteins of HPV 18 and 45, 11, 56, 58, 59 and 70.

### Example 3

### Immunohistochemical detection of HPV E7 protein in cervical cancer biopsies

To determine the HPV status in cervical biopsies from 30 patients, first, PCR analysis was used to determine the different HPV genotypes. 26 (87 %) of the biopsies were HPV-DNA positive, nine (30 %) contained only HPV 16 DNA and three (10 %) only HPV 18 DNA. Eight (23 %) were HPV 16 and 18 positive and six (23 %) contained HPV 16, 18 and other HPV types. In the remaining four biopsies the HPV type was apparently not detectable by the PCR analysis. As negative controls, 22 cervical biopsies containing normal, squamous and glandular epithelia were used. To test, whether the anti-HPV 16 E7 and anti-HPV 18 E7 monoclonal rabbit antibodies according to the invention could detect the HPV E7 proteins in paraffin sections of these biopsies the following protocol for immunohistochemistry was employed.

Immunohistochemistry was performed on paraffin-embedded tissue sections derived from cervical biopsies. 2 µm sections were mounted on slides, deparaffinized in xylene (2 x 12 minutes), rehydrated and processed for antigen retrieval by treatment for 1 hour in a steamer in Target Retrieval Solution (DAKO S1700) for the detection of HPV E7. Endogenous peroxidase activity was blocked by incubation in 20 % H₂O₂/methanol for 30 minutes. Sections were washed and incubated for 15 minutes in blocking buffer (10 % goat or rabbit serum from DAKOCytomation Germany, respectively, 5 % BSA in 1xTris buffer). Blocking solution was removed. The sections were either incubated with the biotinylated monoclonal rabbit anti-HPV 16 E7 or 18 E7 antibodies of the present invention (at appropriate dilutions) for 1 h at room temperature in 5 % BSA/1x TBS in a wet chamber. Samples were rinsed in 1 x Tris/0.1 % Tween20 and incubated with secondary IgGs (DAKOCytomation, Germany) for 45 min at room temperature in a wet chamber. After washing, samples were incubated with streptavidin peroxidase conjugate (Sigma, Vienna) for 30 minutes at room temperature. Bound antibodies were visualized with DAB solution (Sigma, Vienna) as substrate. Counterstaining was performed with Hemalaun (Merck, Vienna). The specimens were dehydrated, and mounted using Entellan (Merck, Vienna). Brightfield microscopy with photography was performed using Olympus CH30 microscope and a Sony DSC-W15 Cyber-shot camera.

In all 23 biopsies tested positive for HPV 16 DNA by the PCR analysis, the anti-HPV 16 E7 antibody of the present invention recognised almost all epithelial tumor cells within the tumor islets but stained no cells in adjacent connective tissues. No HPV 16 E7 protein was detected in normal cervical specimens, neither in connective tissue and cervical glandular epithelia nor in normal cervical squamous epithelia.

To determine whether the HPV 18 E7 protein could be detected by the anti-HPV 18 E7 antibody of the present invention immunohistochemistry experiments were conducted using the biopsies tested positive for HPV 18 DNA by the PCR analysis. Similar to the anti-HPV 16 E7 antibody, the anti-HPV 18 E7 antibody stained almost all tumor cells within the respective tumors, but did not stain cells in the adjacent connective tissue. No staining was detected in normal cervical specimens.

Surprisingly, the anti-HPV 16 E7 as well as the anti-HPV 18 E7 antibody of the present invention could detect the respective protein in the four biopsies in which HPV DNA was undetectable by the PCR analysis, suggesting that the cells of these biopsies were indeed HPV infected which was not detected by the PCR analysis.

### Example 4

### Detection of high-risk HPV 16 E7 proteins in liquid-based cytology

Based on results obtained with paraffin sections, it was tested whether the anti-HPV 16 E7 antibody according to the present invention can be used in liquid-based cytology (LBC). To establish the procedure, LBC samples from healthy women were spiked with CaSki cells (HPV 16 positive), and U-20S cells (HPV negative) and processed according to the following protocol:
CaSki cells and U-20S cells were harvested and resuspended in ThinPrep-Buffer (Cytyc SA, Geneva, Switzerland). Cervical swabs were resuspended in ThinPrep-Buffer, mixed with the cultured cells,
and 10 to 100 µl of the mixture were dropped with a pipette on a ThinPrep-glass slide (Cytyc SA, Geneva, Switzerland). The cells were allowed to dry over night at room temperature. Cells were fixed using 4% PFA at RT for 30 min in a cuvette, and washed by gentle shaking 2 x 5 min at RT in TBS + 0.05% Tween20. For antigen retrieval, cells were incubated with 10 mM NaCitrate pH 6.0 + 0.3% TritonX100 for 15 min at RT in a cuvette, washed 2 x 5 min at RT in TBS + 0.05% Tween20. After peroxidase blocking, slides were incubated for 30 min with 200 µl normal goat serum (DakoCytomation) in a wet chamber (1:10 dilution in 1 % BSA-TBS + 0.05%Tween20).
Each slide was incubated with 200 µl primary antibody at appropriate dilution in 1% BSA-TBS + 0.05% Tween20 for 1 hour at RT in a wet chamber. Slides were washed and incubated with biotinylated secondary antibody (DakoCytomation) at appropriate dilution in 1 % BSA-TBS + 0,05 % Tween20 for 1 hour at RT. Slides were washed and incubated with 200 µl Peroxidase Conjugate (Sigma; 1:500 dilution in 1 % BSA-TBS + 0.05% Tween20) in a wet chamber, and subsequently rinsed in distilled water. For visualization, peroxidase Substrate Kit SK-4200AEC-Kit (Vector, Vienna, Austria) was used.
Haematoxylin served as counterstain.

This procedure yielded a very specific staining of the HPV 16 positive CaSki cells with the anti-HPV 16 E7 antibody of the present invention, whereas neither cervical cells from healthy probands, nor U-2OS cells were stained, suggesting a very high specificity of the antibody (see figure 6).

### Example 5

### ELISA-based HPV diagnosis

### Cell lysis

Cells from cell cultures were washed with PBS and resuspended in ice-cold lysis buffer (PBS- 0.1 % Tween, containing 1 complete EDTA-free protease inhibitor cocktail tablet (Roche) per 50 ml lysis buffer). Lysates were frozen at -80 °C, thawed at room temperature, and centrifuged (4°C, 13,000 rpm, 20 min). Supernatants were processed in the ELISA procedure.

In the case of clinical samples, cervical smears were collected from the cervix using a brush. The brush was placed immediately into the collecting tube containing 1 ml lysis buffer without protease inhibitors (PBS- 0.1 % Tween20). After the stick of the brush was shortened and the collecting tube was closed, the sample was stored immediately at -80°C until use. The sample was thawed at room temperature and immediately 20 µl of 50x protease inhibitor stock solution (complete EDTA-free, Roche) per collecting tube was added. After resuspending the remaining sample on the brush by mixing it in the lysis buffer, the remaining liquid from the brush was wiped off at the edge of the tube and the brush was discarded. The sample was centrifuged at 4°C, 13,000 rpm for 20 min. Supernatants were processed in the ELISA procedure.

### ELISA protocol

100 µl coating buffer (0.1 M NaHCO₃, pH 9.6) containing a mixture of goat polyclonal anti-high-risk HPV antibodies was added to each well of a 96 well plate (F96 Maxisorp Nunc-Immuno Plate), and incubated overnight at 4°C. Wells were washed 3x with washing buffer (PBS, 0.05 % Tween 20; pH 7.4); 300 µl blocking buffer (Universal Casein Diluent/Blocker, SDT) was added to each well, and incubated for 2 hours at room temperature. Wells were washed 3x with washing buffer, afterwards recombinant E7 protein or cell lysate (200 µl; diluted in lysis buffer: PBS, 0.1 % Tween, protease inhibitors) was added and incubated for 30 minutes at room temperature. Wells were aspirated and again 200 µl protein or cell lysate were added and incubated for 30 minutes at room temperature, Wells were washed 3x with washing buffer. 100 µl monoclonal detection antibody (anti-HPV 16 E7 or anti-HPV 18 E7 antibodies of the present invention) in its appropriate dilution in Universal Casein Diluent/Blocker were added to each well, incubated for 1 hour at room temperature, and washed 3x with washing buffer. 100 µl SA-PolyHRP-conjugate (SDT) in its appropriate dilution in Universal Casein Diluent/Blocker were added to each well and incubated for 1 hour at room temperature. Afterwards wells were washed 6x with washing buffer. 100 µl detection reagent (es(HS)TMB, SDT) were added to each well, incubated for 30min in the dark at room temperature. 50 µl stop solution (2N H₂SO₄) were added to each well, and absorbance (450 nm) determined by an ELISA reader.

### Results

To determine the sensitivity of the monoclonal detection antibodies of the present invention decreasing amounts of the respective recombinant E7 protein were tested with the ELISA protocol. In this setting, 10 pg of HPV 16 E7 was easily detectable by the monoclonal anti-HPV 16 E7 antibody and similar sensitivity was obtained for the HPV 18 E7 antibody with HPV 18 E7 and HPV 45 E7 proteins (see figure 1).

When the monoclonal detection antibodies of the present invention were biotinylated and incubated with streptavidin-poly-HRP-conjugate instead of a secondary antibody, an additional signal amplification could be achieved. In this optimised setting, the specific detection limit of the monoclonal antibodies according to the present invention was reduced to 500 fg ― 1 pg in case of HPV 16 E7 and to 250 fg ― 500 fg for HPV 18 E7 protein (see figure 2).

With this setting it could also be shown that proteins of low-risk HPVs like HPV 6 or HPV 11 are not detected by the anti-HPV 16 E7 or the anti-HPV 18 E7 antibody of the present invention (see figure 3).

To determine, whether this ELISA format is suitable for the detection of E7 proteins in tumor cells, HPV negative cells (U-20S) were mixed with decreasing amounts of HeLa cells (HPV 18 positive) and CaSki cells (HPV 16 positive), respectively. Cell mixtures were lysed and analyzed by ELISA (see above). These experiments revealed that 2,500 - 5,000 CaSki cells as well as 500 - 1,000 HeLa cells (in the background of HPV negative U-20S cells) can be detected by the respective antibody of the present invention (see figure 4).

To determine, whether E7 proteins could also be detected in clinical samples with this setting, cervical smears were tested. For this purpose, two consecutive Pap Smears were taken from patients or healthy probands. One sample was used for cytological classification according to Papanicolaou and subsequent determination of the HPV subtype by PCR analysis. The second sample was processed for the ELISA protocol (see above). In total, five samples were processed (one HPV 16 positive and three HPV negative), yielding clear signals above background using the antibodies according to the present invention for the HPV 16 and 18 positive samples, whereas the HPV negative samples did not produce a detectable signal. These data show that the sensitivity of the present assay employing the antibodies of the present invention is high enough to allow reliable detection of high-risk E7 proteins in clinical samples and thereby enabling reliable diagnosis of high-risk HPV infections.

## Claims

1. A monoclonal anti-HPV (human papillomavirus) E7 antibody, which is capable of specifically recognising an epitope of the C-terminal region of a HPV E7 protein.

2. The monoclonal antibody according to claim 1, wherein the epitope is an epitope of the zinc finger domain of the C-terminal region of the HPV E7 protein.

3. The monoclonal antibody according to any one of the foregoing claims, wherein the epitope is a conformational epitope.

4. The monoclonal antibody according to any one of the foregoing claims, wherein the recognised E7 protein is the E7 protein of HPV 16 subtypes or HPV 18 subtypes.

5. The monoclonal antibody according to claim 4, wherein the epitope comprises the amino acid sequence set forth in SEQ ID No. 1, 2, 3 or 4.

6. A hybridoma cell line, capable of producing the monoclonal antibody according to any one of the foregoing claims.

7. The hybridoma cell line of claim 6, which has the accession no. DSM ACC3034.

8. A monoclonal antibody, obtainable from the supernatant of the hybridoma cell line according to claims 6 and 7.

9. A monoclonal anti-HPV E7 antibody, which is capable of specifically recognising an epitope of the N-terminal region of a human papillomavirus E7 protein.

10. The monoclonal antibody according to claim 9, wherein the epitope has the amino acid sequence set forth in SEQ ID No. 5.

11. A hybridoma cell line, capable of producing the monoclonal antibody according to claims 9 and 10.

12. The hybridoma cell line, which has the accession no. DSM ACC3035.

13. A monoclonal antibody, obtainable from the supernatant of the hybridoma cell line according to claims 11 and 12.

14. The monoclonal antibody according to any one of claims 1 to 5, 8 to 10 or 13, wherein said antibody is labelled with a radioactive, enzymatic or fluorescent group.

15. The monoclonal antibody according to any one of claims 1 to 5, 8 to 10, 13 or 14, which is a rabbit antibody.

16. A diagnostic composition, comprising the monoclonal antibody according to any one of claims 1 to 5, 8 to 10 or 13 to 15 as active agent.

17. A diagnostic kit, comprising the monoclonal antibody according to claims 1 to 5, 8 to 10 or 13 to 15 as a first agent and antibodies directed against HPV E7 proteins as a second agent.

18. An in vitro method for the immunological, preferably immunohistochemical or immunofluorescence-based diagnosis of HPV infections, comprising
a) incubating a biological sample with the monoclonal antibody according to claims 1 to 5, 8 to 10 or 13 to 15 and
b) detecting HPV E7 protein in the biological sample by detecting the antibody specifically bound to the E7 protein and thereby allowing the diagnosis of a HPV infection.

19. An in vitro method for the ELISA-based diagnosis of HPV infections, comprising
aa) coating a support with capture antibodies directed against HPV E7 proteins,
bb) adding a biological sample to the coated support,
cc) incubating the support with the monoclonal detection antibody according to any one of claims 1 to 5, 8 to 10 or 13 to 15,
dd) identifying HPV E7 protein specifically bound by the capture antibodies by detecting the monoclonal detection antibody specifically bound to the E7 protein and thereby allowing the diagnosis of a HPV infection.

20. Use of the monoclonal antibody according to any one of claims 1 to 5, 8 to 10 or 13 to 15 for the preparation of a diagnostic composition for the in vitro detection of HPV E7 protein in a biological sample.

21. A method for the preparation of a diagnostic composition, comprising the step of formulating the monoclonal antibody according to any one of claims 1 to 5, 8 to 10 or 13 to 15 with an acceptable carrier, diluent, buffer or storage solution.
